# EUROPÄISCHE PATENTSCHRIFT

(11) **EP 1 224 002 B1**
(45) Veröffentlichungstag und Bekanntmachung des Hinweises auf die Patenterteilung: **12.03.2003**
(21) Anmeldenummer: 99974132.5
(22) Anmeldetag: 26.10.1999
(51) Int. Cl.: A61M 5/32

(54) **INJEKTIONSNADEL-UMHÜLLUNGSVORRICHTUNG**
SHEATHING DEVICE FOR AN INJECTION NEEDLE
DISPOSITIF DE GAINAGE POUR AIGUILLE D'INJECTION

(43) Veröffentlichungstag der Anmeldung: 24.07.2002
(73) Patentinhaber: Rigter, Bart, 88456 Muttensweiler (DE)
(72) Erfinder: Rigter, Bart, 88456 Muttensweiler (DE)
(74) Vertreter: Kiessling, Christian
(86) Internationale Anmeldenummer: DE9903348
(87) Internationale Veröffentlichungsnummer: WO01030426

(56) Entgegenhaltungen:
- US-A- 3 299 891
- US-A- 5 011 479
- US-A- 5 478 315
- US-A- 5 603 699
- US-A- 5 693 022

## Beschreibung

Die Erfindung betrifft eine Vorrichtung zum Umhüllen einer mit einer Injektionsspritze verbundenen Injektionsnadel.

Derartige Vorrichtungen sind z.B. in der US-A-5 011 479 und der US-A-5 693 022 offenbart.

Der Anblick von mit Injektionsnadeln versehenen Injektionsspritzen ist insbesondere für Babys und Kleinkinder belastend und Angst einflößend, da mit einem Einstich der Injektionsnadel in eigenes Körpergewebe Schmerzen vorhergesehen werden. Insbesondere zum Zweck von Impfungen können jedoch so dünne Injektionsnadeln zum Einstechen in menschliches Gewebe Verwendung finden, daß ein größerer Schmerz objektiv nicht mehr empfindbar ist. In solchen Fällen bildet die Angst vor der Spritze objektiv die einzige negative Empfindung, die im Zusammenhang mit einer Injektion auftritt.

Die US-A-3 299 891 schlägt diesbezüglich vor, Spritzen mit speziell für Kinder attraktiven Gehäusen in Tierform auszustatten und damit zu tarnen.

Aufgabe der Erfindung ist es, eine Vorrichtung zu schaffen, mit der der Anblick einer Injektionsnadel bis unmittelbar vor einen Einstechvorgang in menschliches Gewebe verhüllt ist, so daß zum einen einem Kleinkind oder Säugling die Angst vor einem Spritzvorgang genommen ist und andererseits die Injektionsnadel bis zu einem Zeitpunkt unmittelbar vor dem Einstechen in menschliches Gewebe vor Verunreinigungen geschützt ist.

Erfindungsgemäß wird diese Aufgabe gelöst durch eine Vorrichtung zum Umhüllen einer mit einer Injektionsspritze verbundenen Injektionsnadel gemäß Anspruch 1.

Bevorzugte Ausführungsformen der Erfindung sind Gegenstand der Unteransprüche.

Gemäß einer bevorzugten Ausführungsform der erfindungsgemäßen Vorrichtung sind die Klappeneinrichtungen beweglich mit der Befestigungseinrichtung verbunden. Die Klappeneinrichtungen können dabei über Scharniere mit der Befestigungseinrichtung verbunden sein, wobei die Scharniere vorzugsweise in der Befestigungseinrichtung gelagert sind. Eine Beweglichkeit der Klappeneinrichtungen bezüglich der Befestigungseinrichtung ist dadurch auf robuste wie auch auf einfache Weise sichergestellt.

Die Klappeneinrichtungen der erfindungsgemäßen Vorrichtung weisen vorzugsweise jeweils aneinander angrenzende drehkraftübertragende Bereiche auf, die bewirken, daß eine Drehbewegung einer Klappeneinrichtung eine Drehbewegung bei einer anderen Klappeneinrichtung bewirkt. Auf diese Weise ist sichergestellt, daß sich die Klappeneinrichtungen der erfindungsgemäßen Vorrichtung jeweils mit gleicher Auslenkung und in jeweils gleiche Richtung bewegen. Die drehkraftübertragenden Bereiche der Klappeneinrichtungen sind dabei vorzugsweise als Zahnkränze und/oder Zahnstangen ausgebildet und dabei insbesondere einstückig mit der jeweiligen Klappeneinrichtung ausgebildet.

Um eine Auslenkung der Klappeneinrichtungen auf leichte Weise durch Handbetätigung zu ermöglichen, ist an mindestens einer Klappeneinrichtung ein Hebel zum Ausüben einer Drehbewegung von Hand vorgesehen.

Die Klappeneinrichtungen der erfindungsgemäßen Vorrichtung können alternativ zu Scharnieren über weichelastisch ausgebildete Verbindungsteile mit der Befestigungseinrichtung verbunden sein. Die Klappeneinrichtungen können dabei insbesondere insgesamt aus einem weichelastischen Material hergestellt sein. Die Elastizität und die Vorspannung des weichelastischen Materials sind dabei so vorgesehen, daß die Klappeneinrichtungen ohne Krafteinwirkung von außen in einem Grundzustand vorliegen, in dem sie eine Injektionsnadel umhüllen, wobei sie durch ein leichtes Aufbringen entsprechender Druckkräfte insbesondere von Hand in einen Zustand überführbar sind, in dem die Injektionsnadel freiliegt.

Die Befestigungseinrichtung der erfindungsgemäßen Vorrichtung ist vorzugsweise als Kopf eines Tieres oder eines Menschen ausgebildet, wobei die Klappeneinrichtungen dabei insbesondere als Maul eines Tieres oder eines Menschen ausgebildet sind. Das Tier ist dabei vorzugsweise als Krokodil ausgebildet. Der Mensch ist dabei vorzugsweise als Clown ausgebildet. Die Befestigungseinrichtung kann dabei auch in Form eines beliebigen Gegenstandes ausgebildet sein, beispielsweise in Form'eines Autos, wobei die Motorhaube des Autos eine Klappeneinrichtung bildet.

Die erfindungsgemäße Vorrichtung wird im folgenden anhand einer bevorzugten Ausführungsform erläutert, die in den Figuren der Zeichnung dargestellt ist. Darin zeigen:
- Fig.1: eine bevorzugte Ausführungsform der erfindungsgemäßen Injektionsnadel-Umhüllungsvorrichtung in einer Seitenansicht, in einem ersten, die Injektionsnadel umhüllenden Zustand;
- Fig.2: die in Fig. 1 dargestellte Ausführungsform der erfindungsgemäßen Injektionsnadel-Umhüllungsvorrichtung in einer Seitenansicht, in einem zweiten, die Injektionsnadel freigebenden Zustand;
- Fig.3: die Klappeneinrichtungen der in den Figuren 1 und 2 dargestellten Ausführungsformen der erfindungsgemäßen Injektionsnadel-Umhüllungsvorrichtung in einem Längsschnitt;
- Fig.4: einen Ausschnitt der in Fig. 1 dargestellten Ausführungsform der erfindungsgemäßen Injektionsnadel-Umhüllungsvorrichtung, in einer Ansicht von oben;
- Fig.5: einen Ausschnitt der in Fig. 1 dargestellten Ausführungsform der erfindungsgemäßen Injektionsnadel-Umhüllungsvorrichtung, in einer Ansicht von unten.

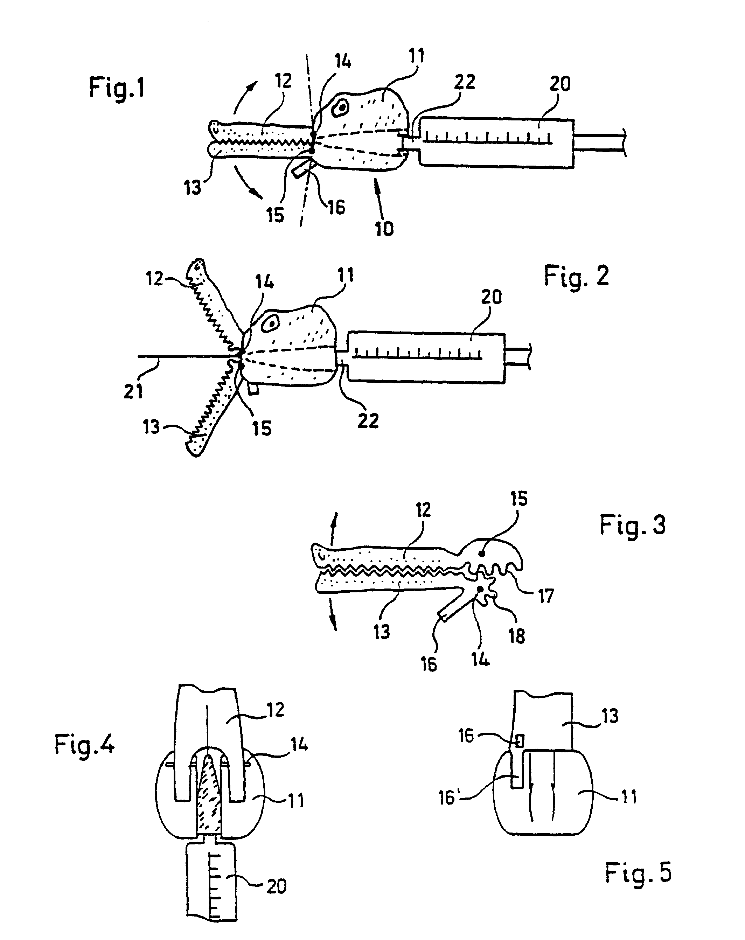

Bei der in den Figuren 1 und 2 dargestellten Ausführungsformen der erfindungsgemäßen Injektionsnadel-Umhüllungsvorrichtung 10 ist eine Befestigungseinrichtung 11 mit einer Ansatzbuchse einer Injektionsspritze 20 verbunden, wobei an der Ansatzbuchse 22 eine Injektionsnadel 21 befestigt ist. Die Befestigungseinrichtung 11 weist an ihrem der Injektionsspritze 20 fernen Ende zwei Klappeneinrichtungen 12, 13 auf, die jeweils über Scharniere 14, 15 drehbar in der Befestigungseinrichtung 11 gelagert sind. Die untere Klappeneinrichtung 13 weist dabei einen Hebel 16 auf, der von Hand betätigbar ist, um die Klappeneinrichtungen 12, 13 aus einem ersten Zustand, in dem sie, wie in Fig. 1 dargestellt, die Injektionsnadel 21 umhüllen, in einen zweiten Zustand überführbar sind, in dem sie, wie in Fig. 2 dargestellt, die Injektionsnadel 21 freigeben.

Wie in Fig. 3 dargestellt, weisen die Klappeneinrichtungen 12, 13 im Bereich der Scharniere 14, 15 einen Zahnkranz 18 sowie eine Zahnstange 17 auf, die als drehkraftübertragende Bereiche wirken und bewirken, daß eine Drehbewegung einer Klappeneinrichtung (13) eine Drehbewegung bei der anderen Klappeneinrichtung (12) bewirkt.

In Figur 4 ist die Lagerung der oberen Klappeneinrichtung 12 in dem Scharnier 14 der Befestigungseinrichtung 11 erkennbar.

Aus Figur 5 ist erkennbar, daß die Befestigungseinrichtung 11 in ihrem unteren Bereich eine dem Hebel 16 gegenüberstehende Aussparung 16' aufweist, die einen Freiraum bildet, in den der Hebel 16 von Hand bewegbar ist, um die untere Klappeneinrichtung 13 in einen Zustand zu überführen, in dem die Injektionsnadel 21 freiliegt.

Das oben erläuterte Ausführungsbeispiel der Erfindung dient lediglich dem Zweck eines besseren Verständnisses der durch die Ansprüche vorgegebenen erfindungsgemäßen Lehre, die als solche durch das Ausführungsbeispiel nicht eingeschränkt ist.

## Patentansprüche

1. Vorrichtung (10) zum Umhüllen einer mit einer Injektionsspritze (20) verbundenen Injektionsnadel (21), mit einer Befestigungseinrichtung (11) zum Befestigen der Vorrichtung an der Injektionsspritze sowie mindestens zwei Klappeneinrichtungen (12, 13), die reziprozierbar aus einem Zustand, in dem die Injektionsnadel umhüllt ist, überführbar sind in einen Zustand, in dem die Injektionsnadel freiliegt, wobei die Klappeneinrichtungen (12, 13) jeweils aneinander angrenzende drehkraftübertragende Bereiche (17, 18) aufweisen, die bewirken, dass eine Drehbewegung einer Klappeneinrichtung eine Drehbewegung bei einer anderen Klappeneinrichtung bewirkt, **dadurch gekennzeichnet, dass** eine der Klappeneinrichtungen (13) einen an einen der drehkraftübertragende Bereiche angrenzenden Hebel (16) aufweist, der von Hand betätigbar ist, um die Klappeneinrichtungen (12, 13) aus einem ersten Zustand, in dem sie die Injektionsnadel umhüllen, in einen Zustand zu überführen, in dem die Injektionsnadel (21) freiliegt, und die Befestigungseinrichtung (11) in ihrem unteren Bereich eine dem Hebel gegenüberstehende Aussparung (16') aufweist, die einen Freiraum bildet, in den der Hebel (16) von Hand bewegbar ist.

2. Vorrichtung nach Anspruch 1, **dadurch gekennzeichnet, daß** die Klappeneinrichtungen (12, 13) beweglich mit der Befestigungseinrichtung (11) verbunden sind.

3. Vorrichtung nach Anspruch 2, **dadurch gekennzeichnet, daß** die Klappeneinrichtungen (12, 13) über Scharniere mit der Befestigungseinrichtung (11) verbunden sind.

4. Vorrichtung nach Anspruch 3, **dadurch gekennzeichnet, daß** die Scharniere in der Befestigungseinrichtung (11) gelagert sind.

5. Vorrichtung nach einem der vorhergehenden Ansprüche, **dadurch gekennzeichnet, daß** die Klappeneinrichtungen (12, 13) jeweils aneinander angrenzende drehkraftübertragende Bereiche (17, 18) aufweisen, die bewirken, daß eine Drehbewegung einer Klappeneinrichtung eine Drehbewegung bei einer anderen Klappeneinrichtung bewirkt.

6. Vorrichtung nach Anspruch 5, **dadurch gekennzeichnet, daß** die drehkraftübertragenden Bereiche (17,18) als Zahnkränze und/oder Zahnstangen ausgebildet sind.

7. Vorrichtung nach einem der Ansprüche 5 oder 6, **dadurch gekennzeichnet, daß** die drehkraftübertragenden Bereiche (17, 18) einstückig mit den jeweiligen Klappeneinrichtungen (12, 13) ausgebildet sind.

8. Vorrichtung nach einem der vorhergehenden Ansprüche, **dadurch gekennzeichnet, daß** an mindestens einer Klappeneinrichtung (13) ein Hebel (16) zum Ausüben einer Drehbewegung von Hand vorgesehen ist.

9. Vorrichtung nach einem der Ansprüche 1 oder 2 **dadurch gekennzeichnet, daß** die Klappeneinrichtungen (12, 13) über weichelastisch ausgebildete Verbindungsstücke mit der Befestigungseinrichtung verbunden sind.

10. Vorrichtung nach einem der Ansprüche 1 oder 2, **dadurch gekennzeichnet, daß** die Klappeneinrichtungen (12, 13) insgesamt aus einem weichelastischen Material hergestellt sind.

11. Vorrichtung nach einem der vorhergehenden Ansprüche, **dadurch gekennzeichnet, daß** die Befestigungseinrichtung (11) als Kopf eines Tieres oder eines Menschen oder als ein Auto ausgebildet ist.

12. Vorrichtung nach einem der vorhergehenden Ansprüche, **dadurch gekennzeichnet, daß** die Klappeneinrichtungen (12, 13) als Maul eines Tieres oder eines Menschen ausgebildet sind.

13. Vorrichtung nach einem der Ansprüche 11 oder 12, **dadurch gekennzeichnet, daß** das Tier ein Krokodil ist.

14. Vorrichtung nach einem der Ansprüche 11 oder 12, **dadurch gekennzeichnet, daß** der Mensch ein Clown ist.

## Claims

1. Apparatus (10) for covering an injection needle (21) connected to an injection syringe (20), having a fastening means (11) for fastening the apparatus to the injection syringe and at least two flap means (12, 13) which are reciprocatably transferable from a first state, in which the injection needle is covered, into a second state, in which the injection needle is uncovered, wherein the flap means (12, 13) are each provided with spin-transferring areas (17, 18) having the effect that a twisting movement of one flap means causes a twisting movement of the other flap means, **characterized in that** one of the flap means (13) has a lever (16) connected to one of the spin-transferring areas in order to transfer the flap means (12, 13) from a first state, in which the injection needle is covered, into a second state, in which the injection needle (21) is uncovered, the fastening means (11) being provided in its lower part opposite to the lever with an indentation forming a free space into which the lever (16) is movable by hand.

2. Apparatus according to claim 1, **characterized in that** the flap means (12, 13) are movably connected to the fastening means (11).

3. Apparatus according to claim 2, **characterized in that** the flap means (12, 13) are connected to the fastening means (11) via hinges.

4. Apparatus according to claim 3, **characterized in that** the hinges are journaled in the fastening means (11).

5. Apparatus according to one of the previous claims, **characterized in that** the flap means (12, 13) are each provided with mutually abutting spin-transferring areas (17, 18), having the effect that a twisting movement of one flap means causes a twisting movement of the other flap means.

6. Apparatus according to claim 5, **characterized in that** the spin-transferring areas (17, 18) are embodied as cogwheels and/or cog racks.

7. Apparatus according to one of claims 5 or 6, **characterized in that** the spin-transferring areas (17, 18) are embodied integrally with the corresponding flap means (12, 13).

8. Apparatus according to one of the previous claims, **characterized in that** on at least one flap means (13) a lever (16) is provided for excerpting a twisting movement by hand.

9. Apparatus according to one of claims 1 or 2, **characterized in that** the flap means (12, 13) is connected to the fastening means via soft resilient connecting units.

10. Apparatus according to one of claims 1 or 2, **characterized in that** the flap means (12, 13) are generally provided of a soft resilient material.

11. Apparatus according to one of the previous claims, **characterized in that** the fastening means (11) is embodied as the head of an animal or a human or a car.

12. Apparatus according to one of the previous claims, **characterized in that** the flap means (12, 13) is embodied as the mouth of an animal or a human.

13. Apparatus according to one of claims 11 or 12, **characterized in that** the animal is a crocodile.

14. Apparatus according to one of claims 11 or 12, **characterized in that** the human is a clown.

## Revendications

1. Dispositif (10) d'enrobage d'une aiguille d'injection (21) attachée à une seringue injectrice (20), comportant un élément de fixation (11) pour fixer le dispositif sur la seringue injectrice et au moins deux éléments clapets (12, 13) qui dans un mouvement de va et vient peuvent être passés d'un état où l'aiguille d'injection (21) est enveloppée à un état où l'aiguille d'injection (21) est exposée, les éléments clapets (12, 13) présentant des zones adjacentes de transmission de force de rotation respectives (17, 18) qui ont pour effet que la rotation de l'un des éléments clapets provoque une rotation de l'autre des éléments clapets, **caractérisé en ce que** l'un des éléments clapets (13) présente un levier (16) adjacent à l'une des zones de transmission de force de rotation, qui peut être actionné à la main pour passer les éléments clapets (12, 13) d'un premier état, où ils enveloppent l'aiguille d'injection (21), à un deuxième état, où l'aiguille d'injection (21) est exposée, et que l'élément de fixation (11) présente, dans sa partie inférieure, un évidement (16') opposé au levier (16) et constituant un espace libre dans lequel le levier 16 peut être poussé à la main.

2. Dispositif selon la revendication 1, **caractérisé en ce que** les éléments clapets (12, 13) sont reliés à l'élément de fixation (11) de façon mobile.

3. Dispositif selon la revendication 2, **caractérisé en ce que** les éléments clapets (12, 13) sont reliés à l'élément de fixation (11) au moyen de charnières.

4. Dispositif selon la revendication 3, **caractérisé en ce que** les charnières sont logées dans l'élément de fixation (11).

5. Dispositif selon l'une quelconque des revendications précédentes, **caractérisé en ce que** les éléments clapets (12, 13) présentant des zones adjacentes de transmission de force de rotation respectives (17, 18) qui ont pour effet que la rotation de l'un des éléments clapets provoque une rotation de l'autre des éléments clapets.

6. Dispositif selon la revendication 5, **caractérisé en ce que** les zones de transmission de force de rotation (17, 18) sont réalisées sous forme de couronnes dentées et/ou de crémaillères.

7. Dispositif selon l'une des revendications 5 ou 6, **caractérisé en ce que** les zones de transmission de force de rotation (17, 18) sont réalisées en une pièce avec les éléments clapet (12, 13) correspondants.

8. Dispositif selon l'une quelconque des revendications précédentes, **caractérisé en ce qu'** un levier (16) pour exécuter une rotation à la main est prévu sur au moins l'un (13) des éléments clapets.

9. Dispositif selon l'une des revendications 1 ou 2, **caractérisé en ce que** les éléments clapets (12, 13) sont attachées à l'élément de fixation (11) au moyen d'attaches souples.

10. Dispositif selon l'une des revendications 1 ou 2, **caractérisé en ce que** les éléments clapets (12, 13) entiers sont réalisés en une matière souple.

11. Dispositif selon l'une quelconque des revendications précédentes, **caractérisé en ce que** l'élément de fixation (11) a la forme de la tête d'un animal ou d'un homme ou la forme d'une voiture.

12. Dispositif selon l'une quelconque des revendications précédentes, **caractérisé en ce que** les éléments clapets (12, 13) sont réalisés en forme de bouche d'un animal ou d'un homme.

13. Dispositif selon l'une des revendications 11 ou 12, **caractérisé en ce que** l'animal est réalisé sous forme d'un crocodile.

14. Dispositif selon l'une des revendications 11 ou 12, **caractérisé en ce que** l'homme est réalisé sous forme d'un clown.
